# EUROPÄISCHE PATENTANMELDUNG

(11) **EP 4 488 377 A1**
(43) Veröffentlichungstag der Anmeldung: **08.01.2025**
(21) Anmeldenummer: 23184069.5
(22) Anmeldetag: 07.07.2023
(51) Int. Cl.: C12P 19/04, C12N 1/20, C12N 1/14, C08J 3/00, C08J 5/00, C08L 1/00

(54) **VERFAHREN ZUM FORMEN EINES CELLULOSEGEWEBES**

(71) Anmelder: Universität Linz, 4040 Linz (AT); Universität für künstlerische und industrielle Gestaltung Linz, 4020 Linz (AT)
(72) Erfinder: Baumgartner, Werner, 4202 Kirchschlag (AT); Weth, Agnes, 97241 Bergtheim-Dipbach (DE); Wurm, Jasmin, 4061 Pasching (AT); Heitz, Johannes, 4040 Linz (AT); Gollob, Emanuel, 1180 Wien (AT); Braumann, Johannes, 5020 Salzburg (AT); Luible-Bär, Christiane, 5161 Elixhausen (AT); Eichinger, Miriam, 4020 Linz (AT)
(74) Vertreter: Burgstaller, Peter

(57) **Zusammenfassung**

Die Erfindung betriff ein Verfahren zur Bildung eines nahtlosen Cellulosegewebes (9), welches die Schritte umfasst,
dass in einem ersten Schritt zumindest eine Cellulose erzeugende Bakterien- und/oder Pilzkultur (4) auf die Oberfläche eines dreidimensionalen Objekts (1) aufgebracht wird,
dass in einem zweiten Schritt das dreidimensionale Objekt (1) in einer geschlossenen Umgebung (3) wiederholt mit einer Nährstofflösung (5) besprüht wird, solange bis die Bakterien- und/oder Pilzkultur (4) eine Celluloseschicht (7) in der gewünschten Dicke abgeschieden hat,
dass in einem dritten Schritt die Celluloseschicht (7) am dreidimensionalen Objekt thermisch bearbeitet wird, um das Cellulosegewebe (9) in stabilisierter Form zu erhalten.

## Beschreibung

Die Erfindung betrifft ein Verfahren zum Formen eines Cellulosegewebes an dreidimensionalen Objekten.

Das gegenständliche Cellulosegewebe wird dabei durch Bakterien und/oder Pilze geformt.

Bakterien, Pilze und Kulturen aus Bakterien und Pilzen, welche Cellulose abscheiden können, sind in der Wissenschaft bekannt.

Nach dem Stand der Technik ist es bekannt, zunächst flächige bzw. zweidimensionale Cellulosegewebe wachsen zu lassen und diese dann nachfolgend, wie in der Textilindustrie üblich, zu vernähen, um Kleidungsstück herzustellen.

Die der Erfindung zu Grunde liegende Aufgabe besteht darin, ein Verfahren bereit zu stellen, welches eine Bildung des Cellulosegewebes an einem dreidimensionalen Objekt ermöglicht.

Für das Lösen der Aufgabe wird ein Verfahren nach Anspruch 1 vorgeschlagen.

In einer Ausführungsvariante wird ein Verfahren zur Bildung eines nahtlosen Cellulosegewebes vorgeschlagen, umfassend die Schritte,
dass in einem ersten Schritt zumindest eine Cellulose erzeugende Bakterien- und/oder Pilzkultur auf die Oberfläche eines dreidimensionalen Objekts aufgebracht wird,
dass in einem zweiten Schritt das dreidimensionale Objekt in einer geschlossenen Umgebung wiederholt mit einer Nährstofflösung besprüht wird, solange bis die Bakterien- und/oder Pilzkultur eine Celluloseschicht in der gewünschten Dicke abgeschieden hat,
dass in einem dritten Schritt die Celluloseschicht am dreidimensionalen Objekt thermisch bearbeitet wird, um das Cellulosegewebe in stabilisierter Form zu erhalten.

Bevorzugt wird in einem vierten Schritt das stabilisierte Cellulosegewebe vom Objekt abgenommen.

Bevorzugt wird das Objekt nachfolgend erneut für die Bildung eines Gewebes gemäß dem gegenständlichen Verfahren verwendet. Das Auftragen der Bakterien- und/oder Pilzkultur auf das dreidimensionale Objekt kann im ersten Schritt durch Besprühen, Bepinseln oder Übergießen erfolgen. Das Auftragen der Bakterien- und/oder Pilzkultur auf das dreidimensionale Objekt kann im ersten Schritt durch Eintauchen des Objekts in eine Flüssigkeit erfolgen. Das Aufbringen kann aber auch durch Auflegen zumindest eines flachen Bakterien- und/oder Pilzkuchens erfolgen. Mehrere Bakterien- und/oder Pilzkuchen können Patch-Work-artig auf das Objekt aufgelegt werden. Das Bakterien- und/oder Pilzwachstum und das damit verbundene Abscheiden von Cellulose erfolgt lateral entlang der Oberfläche und in der Dicke, also senkrecht zur Oberfläche. Durch das laterale Wachstum wird eine geschlossene Celluloseschicht erreicht, auch wenn der Auftrag der Bakterien- und/oder Pilzkulturen nicht lückenlos erfolgt.

In einer Ausführungsvariante werden an unterschiedlichen Bereichen des dreidimensionalen Objekts unterschiedliche Bakterien- und/oder Pilzkulturen aufgebracht. Ein Unterschied kann in der Art bzw. Spezies der Bakterien- und/oder Pilzkulturen liegen. Ein Unterschied kann in der Zusammensetzung zumindest zweier Mischkulturen liegen. Ein Unterschied kann in der Menge der aufgebrachten Kulturen liegen.

Das Aufbringen der Kulturen erfolgt bevorzugt bereits in der geschlossenen Umgebung, in welcher der zweite Schritt des Verfahrens ausgeführt wird.

Als geschlossene Umgebung ist beispielsweise ein Inkubator zu verstehen. Bei der geschlossenen Umgebung kann es sich auch um einen Reinraum handeln.

In der geschlossenen Umgebung herrscht bevorzugt eine hohe Luftfeuchtigkeit von bevorzugt zumindest 90 %.

In der geschlossenen Umgebung liegt ausreichend Sauerstoff vor, beispielsweise in Form von Luft. Es kann eine gefilterte Frischluftzufuhr in die geschlossene Umgebung erfolgen. Es kann eine Sauerstoffzufuhr in die geschlossene Umgebung erfolgen. Auch eine Membran zum Gasaustausch zwischen der geschlossenen Umgebung und der Umgebungsluft ist denkbar.

Das Besprühen mit Nährstofflösung erfolgt in der geschlossenen Umgebung. Beispielsweise erfolgt der zweite Schritt in einem verschließbaren Behälter, wobei die Nährstofflösung ohne Öffnung des Behälters aufgebracht wird. Dies kann durch einen oder mehrere Vernebler, beispielsweise Ultraschallvernebler, erfolgen. Dies kann durch eine oder mehrere Sprühdüsen erfolgen. In einer Ausführungsvariante ist ein Vernebler oder eine Sprühdüse auf einer beweglichen Einheit, beispielsweise einem Roboter, angebracht, um definierte Oberflächenbereiche gezielt besprühen zu können.

Das Besprühen der Oberfläche erfolgt bevorzugt mit geringem Druck und in einem geringen Ausmaß, sodass die Oberfläche des dreidimensionalen Objekts benetzt wird, aber die Kulturen nicht weggespült werden. Insbesondere wird bevorzugt, dass die Nährstofflösung in Form von Tröpfchen an der Oberfläche stehen bleibt und dieses nicht, oder nur in geringem Umfang entlang der Oberfläche nach unten abläuft.

In einer Ausführungsvariante werden unterschiedliche Bereiche des dreidimensionalen Objekts unterschiedlich mit Nährstofflösung versorgt. Ein Unterschied kann insbesondere in der Menge von Nährstofflösung liegen.

Dort wo mehr Nährstofflösung oder über einen längeren Zeitraum Nährstofflösung aufgesprüht wird, wird das Cellulosegewebe dichter und/oder dicker.

Geeignete Nährstofflösungen sind nach dem Stand der Technik bekannt. Die Nährstofflösung enthält jedenfalls Zucker, welcher von den Bakterien und/oder Pilzen unter Bildung von mikrobiell synthetisierter Cellulose umgewandelt wird.

Neben Zucker benötigen die Bakterien und/oder Pilze meist auch Sauerstoff.

Allgemein eignen sich Bakterien und Pilze, welche Cellulosefasern abscheiden. Dabei kann ein einzelnes Bakterium oder ein einzelner Pilz zur Verwendung kommen. Es können aber auch mehrere unterschiedliche Bakterien oder mehrere unterschiedliche Pilze zur Verwendung kommen. Es kann aber auch eine Kombination von zumindest einem Bakterium und einem Pilz zur Verwendung kommen.

Ein geeignetes Bakterium ist beispielsweise *Gluconacetobacter xylinum.* Weitere Beispiele sind Kombucha Kulturen, *Agrobacterium* spp., *Acetobacter* spp., *Azotobacter, Rhizobium* spp., *Sarcina, Alcaligenes* und *Pseudomonas.*

Das dreidimensionale Objekt weist bevorzugt eine strukturierte Oberfläche auf.

Mit der Struktur wird erreicht, dass eine kontrollierte Adhäsion der Kulturen erfolgt. Zudem kann die Struktur zur Flüssigkeitsleitung dienen, bzw. dafür sorgen, dass die aufgesprühten Flüssigkeiten nicht, oder nur auf gewünschte Weise verlaufen.

Die strukturierte Oberfläche kann auch dabei helfen, dass das behandelte Cellulosegewebe besser von der Oberfläche entfernt werden kann, indem die Struktur antiadhäsiv auf die gebildete Cellulosefasern wirkt.

Geeignete Strukturen können durch Prägen, Laserbearbeitung, Gravieren oder andere nach dem Stand der Technik bekannte Methoden hergestellt werden.

Bei den Strukturen handelt es sich bevorzugt um Mikrostrukturen oder Nanostrukturen, also um Strukturen mit Höhenunterschieden im Mikrometerbereich oder Nanometerbereich. Bevorzugt weist die Struktur Höhenunterschiede im Bereich von 50 nm bis 200 µm auf. Als Material des Objekts eignet sich beispielsweise Glas, Kunststoff und Metalle, sofern diese nicht toxisch auf Bakterien oder Pilze wirken.

In einer Ausführungsvariante kann das Objekt oder ein auf das Objekt aufgebrachtes Material mit der abgeschiedenen Cellulose unlösbar verbunden bleiben. Beispielsweise kann es sich beim Objekt um einen vorgefertigten Schuh handeln, welcher im zweiten Schritt des gegenständlichen Verfahrens bereichsweise mit der Celluloseschicht versehen wird.

In einer Ausführungsvariante kann am Objekt, das die gewünschte Form bereitstellt, ein Fasermaterial, einzelne Fasern, ein Gewebe, wie Baumwoll-Gaze, eine Folie oder ein anderes verformbares Material aufgebracht werden, welches von der abgeschiedenen Cellulose überwachsen wird. Alternativ oder zusätzlich ist es auch möglich, zuerst eine Schicht von Cellulosefasern am Objekt wachsen zu lassen und dann eines oder mehrere der oben genannten Materialien aufzulegen, wobei nachfolgend bevorzugt durch die Kulturen weitere Cellulose gebildet wird. Dabei ist es möglich diese Materialien auf unterschiedlichen Ebenen des Cellulosegewebes einzubetten. Dabei kann das am Objekt aufgelegte zusätzliche Material von den Cellulosefasern zumindest teilweise umwachsen werden, sodass ein besonders guter Halt erreicht wird. Das zusätzliche Material kann dazu dienen, um das Cellulosegewebe zu verstärken. Das zusätzliche Material, beispielsweise in Form einer Folie, kann dazu dienen, um das Cellulosegewebe leichter vom Objekt ablösen zu können. Das zusätzliche Material kann derart beschaffen sein, dass dieses nach dem Ablösen vom Objekt vom Cellulosegewebe entfernt werden kann, beispielsweise indem dieses durch eine Substanz und/oder bei Umgebungsbedingungen aufgelöst wird, gegenüber welchen das Cellulosegewebe beständig ist.

In einer anderen Ausführungsvariante können Gegenstände wie beispielsweise Ösen, Knöpfe, Nieten, Bügel, Haken, Verschlüsse, Elektronikkomponenten, RFID-Tags etc... auf das Objekt aufgelegt werden. Damit diese Gegenstände besser in der Cellulose eingeschlossen werden können, können diese ein Stoffband aufweisen, wie dies zum Einnähen von Reißverschlüssen bekannt ist.

Alternativ oder zusätzlich ist es auch möglich zuerst eine Schicht von Cellulosefasern am Objekt wachsen zu lassen und dann einen oder mehrere Gegenstände aufzulegen, wobei nachfolgend bevorzugt durch die Kulturen weitere Cellulose gebildet wird. Dabei ist es möglich Gegenstände auf unterschiedlichen Ebenen des Cellulosegewebes einzubetten.

Das Objekt ist bevorzugt ein formstabiler Körper. Das Objekt kann aber auch aufblasbar sein oder aufblasbare Bereiche umfassen. Das Objekt kann mechanisch verstellbare Bereiche umfassen, beispielsweise um Bereiche des Objekts vom Gewebe wegbewegen zu können. Das Objekt kann aus mehreren Elementen zusammengesetzt sein, sodass einzelne Elemente vom Gewebe getrennt werden können. Das Objekt kann elastisch sein, um das Ablösen des Cellulosegewebe durch Druckeinwirkung und Verformung des Objekts unterstützen zu können. Das Objekt kann zum Ablösen des Cellulosegewebe in Schwingung bzw. Vibration versetzt werden. Das Objekt kann Öffnungen aufweisen, um zum thermischen Behandeln und/oder zum Ablösen des Cellulosegewebes ein Medium, insbesondere Flüssigkeit oder Gas bzw. Luft zwischen das Cellulosegewebe und die Objektoberfläche einbringen zu können. Die Öffnungen können im zweiten Schritt des Verfahrens verschlossen sein.

Wie weiter oben erwähnt, wird im dritten Schritt des Verfahrens das Cellulosegewebe am dreidimensionalen Objekt thermisch bearbeitet, um das Cellulosegewebe zu stabilisieren. Insbesondere erfolgt ein Quervernetzen der Cellulosefasern und die Wasseraufnahmefähigkeit des Cellulosegewebes wird reduziert. Dies erfolgt mit nach dem Stand der Technik bekannten Methoden, mit dem Unterschied, dass diese am dreidimensionalen Objekt und somit auch an der dreidimensionalen Form des Cellulosegewebes vorgenommen werden. Die thermische Bearbeitung umfasst jedenfalls eine Hitzeeinwirkung. Die thermische Bearbeitung umfasst bevorzugt eine Erhitzung des Cellulosegewebes auf eine Temperatur von zumindest 50°C, bevorzugt zumindest 60°. Die Erhitzung kann aber auch auf höhere Temperaturen erfolgen, beispielsweise zumindest 70°C, zumindest 80°C oder zumindest 90°C. Die Erhitzung kann auch zumindest 100°C betragen. Die Erhitzung kann auf eine Temperatur bis an oder über den Siedepunkt von Wasser erfolgen. Die Dauer der Erhitzung kann in Abhängigkeit der Temperatur variieren und über einen Zeitraum erfolgen, um die Bakterien oder Pilze abzutöten, wobei die Dauer von der Höhe der Temperatur und der verwendeten Kultur abhängig ist. Sofern die Werte für Temperatur und Zeitdauer für die verwendete Kultur nicht ohnehin bekannt ist, lässt sich diese am besten durch Versuch ermitteln.

Bevorzugt umfasst die thermische Bearbeitung auch das Aufbringen von Druck.

In einer Ausführungsvarianten erfolgt die thermische Bearbeitung durch Bügeln, also durch Druckausübung auf das Cellulosegewebe durch einen heißen Gegenstand, der über die Oberfläche bewegt wird.

Das thermische Bearbeiten kann das Aufbringen von Flüssigkeiten oder anderen Substanzen umfassen, welche vor, während oder nach der thermischen Bearbeitung auf das Cellulosegewebe aufgebracht werden.

In einer anderen Ausführungsvariante kann das Objekt mit einer Gegenform verpresst werden. Die Gegenform kann in einer Variante aus zwei oder mehr Teilschalen bestehen, welche auf das Objekt zubewegt werden.

Die thermische Bearbeitung kann auch das Kochen und/oder Gerben des Cellulosegewebes umfassen. Beispielsweise kann das Objekt übergossen oder in ein Tauchbad bewegt werden. Eine Behandlung mit Dampf ist auch möglich. Eine Behandlung durch Temperatur- und/oder Druckerhöhung in der geschlossenen Umgebung ist auch möglich.

Durch die thermische Bearbeitung werden die Bakterien und/oder Pilze bevorzugt abgetötet. Bakterien, welche nur getrocknet werden, können bei erneutem Kontakt mit einer Nährstofflösung weiterwachsen.

Durch die Art der thermischen Bearbeitung können die Eigenschaften des resultierenden Materials aus Cellulose beeinflusst werden. Dies ist aus dem Stand der Technik von zweidimensionale gewachsenen Cellulosegeweben bekannt. Alternativ oder zusätzlich können die je nach angedachter Verwendung benötigten Eigenschaften des resultierenden Materials durch Versuch, insbesondere Variierung der thermischen Bearbeitung erreicht und/oder weiter verbessert werden.

Die nach dem Verfahren resultierenden Materialien sind in einer Ausführungsvariante lederähnlich. Diese können in Form oder als Teil von Schuhen, Bekleidung, Accessoires vorliegen. Das lederähnliche Material kann als Alternative zu tierischem Leder dienen.

In einer anderen Ausführungsvariante sind die resultierenden Materialien papierähnlich.

In einer anderen Ausführungsvariante sind die resultierenden Materialien holzähnlich.

Das Material kann während oder nach der thermischen Bearbeitung eingefärbt werden.

Die Erfindung wird an Hand von Zeichnungen veranschaulicht:
- Fig. 1:: Veranschaulicht schematisch ein beispielhaftes dreidimensionales Objekt.
- Fig. 2:: Veranschaulicht schematisch das Aufbringen von Kulturen auf das dreidimensionale Objekt.
- Fig. 3:: Veranschaulicht schematisch das Aufbringen von Nährstofflösung auf das dreidimensionale Objekt.
- Fig. 4:: Veranschaulicht schematisch das dreidimensionale Objekt mit einer gewachsenen Celluloseschicht.
- Fig. 5:: Veranschaulicht schematisch den Beginn der thermischen Behandlung.
- Fig. 6:: Veranschaulicht schematisch den Abschluss der thermischen Behandlung.
- Fig. 7:: Veranschaulicht schematisch das dreidimensionale Objekt und das von diesen abgenommenen Cellulosegewebe.
- Fig. 8:: Veranschaulicht ein Objekt, auf welchem ein Gegenstand platziert ist.
- Fig. 9:: Veranschaulicht ein Objekt, auf welchem ein Material platziert ist.
- Fig. 10:: Veranschaulicht ein Cellulosegewebe, in welchem ein Gegenstand eingewachsen ist.
- Fig. 11:: Veranschaulicht ein Cellulosegewebe, in welchem ein anderes Gewebe eingewachsen ist.

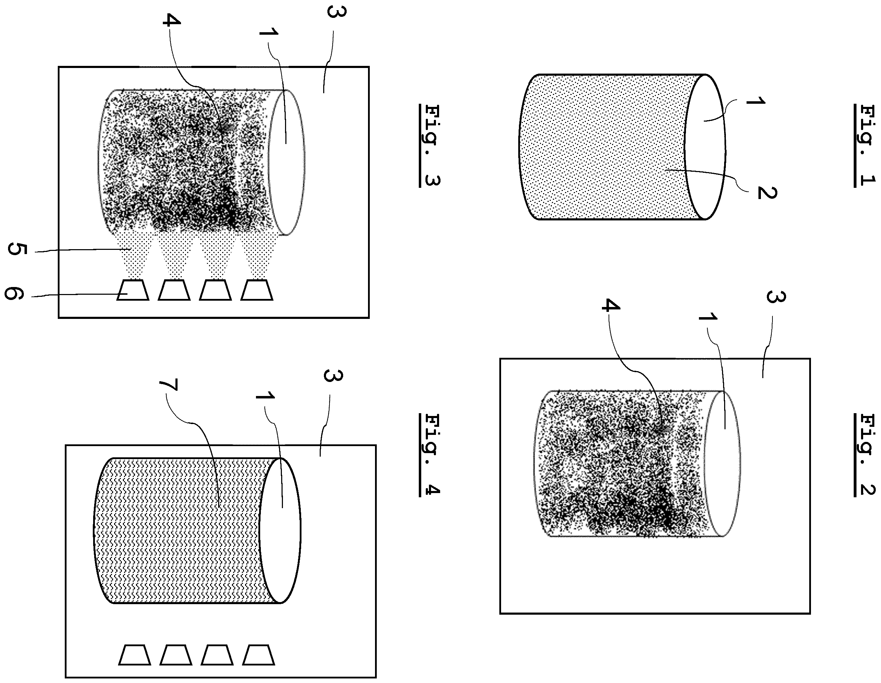

In Fig. 1 ist schematisch ein dreidimensionales Objekt 1 in Form eines Zylinders dargestellt. Das dreidimensionale Objekt 1 kann eine beliebige Form aufweisen. Wie in Fig. 1 dargestellt, wird bevorzugt, dass das Objekt 1 zumindest bereichsweise eine strukturierte Oberfläche 2 aufweist. Das Objekt 1 kann mit einer einheitlichen Struktur versehen sein. Das Objekt 1 kann an unterschiedlichen Stellen auch mit unterschiedlichen Strukturen versehen sein. Das Objekt 1 kann auch Bereiche mit glatter Oberfläche aufweisen. Die Struktur kann in Form eines Musters aus Erhebungen und Vertiefungen vorliegen. Eine Struktur kann auch durch Aufrauen der Oberfläche durch mechanische oder chemische Einwirkung resultieren.

Wie in Fig. 2 veranschaulicht ist, werden in einem ersten Schritt des gegenständlichen Verfahrens Bakterienkulturen und/oder Pilzkulturen 4 auf das Objekt 1, insbesondere auf eine strukturierte Oberfläche 2, aufgebracht. Dies kann bereits in einer geschlossenen Umgebung 3 erfolgen, um eine Verunreinigung, insbesondere durch unerwünschte Schimmelsporen, zu verhindern. Als geschlossene Umgebung 3 kann ein Raum oder ein Behälter dienen, welcher eine ausreichende Abschirmung gegenüber äußeren Einflüssen bietet. Bevorzugt sind die Bedingungen in der geschlossenen Umgebung definierbar, insbesondere indem ein oder mehrere der Parameter Temperatur, Luftfeuchtigkeit, Sauerstoffkonzentration oder Druck einstellbar sind.

Wie in Fig. 3 veranschaulicht ist, sind in der geschlossenen Umgebung 3 bevorzugt Zuführmittel 6 für Nährstofflösung 5 vorhanden. Beispielsweise können die Zuführmittel 6 wie dargestellt in Form von Sprühdüsen vorliegen, welche einen feinen Sprühnebel der Nährstofflösung auf das Objekt 1 richten. Bei der dargestellten Anordnung könnte das Objekt 1 auf einem Drehteller platziert sein, oder die Zuführmittel 6 um das Objekt 1 herumbewegt werden. Alternativ können auch Zuführmittel 6 rund um das Objekt 1 angeordnet sein, um alle Bereiche des Objekts 1 erreichen zu können. Bevorzugt erfolgt das Besprühen automatisiert. Weniger bevorzugt kann das Besprühen auch manuell erfolgen, beispielsweise in einem Handschuhkasten. Wenn Textilien in größerem Maßstab hergestellt werden, können auch mehrere Objekte 1 gemeinsam in einer geschlossenen Umgebung 3 vorliegen und von zumindest einem Roboter, wie einem Portalroboter oder Industrierobotern, besprüht werden. Die Objekte 1 können dabei auf Transportplattformen vorliegen und mittels Schleusen in die und aus der geschlossenen Umgebung 3 bewegt werden.

Das Besprühen der Oberfläche erfolgt wiederholt mit dazwischenliegenden Pausen, sodass die Oberfläche bevorzugt ständig mit Nährstofflösung benetzt ist, aber nicht so viel Nährstofflösung aufgetragen wird, dass diese von der Oberfläche noch unten abläuft. Eine hohe Luftfeuchtigkeit in der geschlossenen Umgebung 3 kann dazu beitragen ein Trocknen der Oberfläche zu verhindern.

Die Versorgung mit Nährstofflösung 5 bewirkt, dass die Kulturen 4 Cellulosefasern abscheiden, welche eine Celluloseschicht 7 bilden. Die Celluloseschicht 7 umgibt das dreidimensionale Objekt 1 bevorzugt in Umfangsrichtung, sodass ein nahtloser geschlossener Cellulosemantel zumindest einen Bereich des Objekts 1 umgibt.

Sobald die Celluloseschicht 7 in der gewünschten Dicke vorliegt, kann die thermische Behandlung der Celluloseschicht 7 erfolgen.

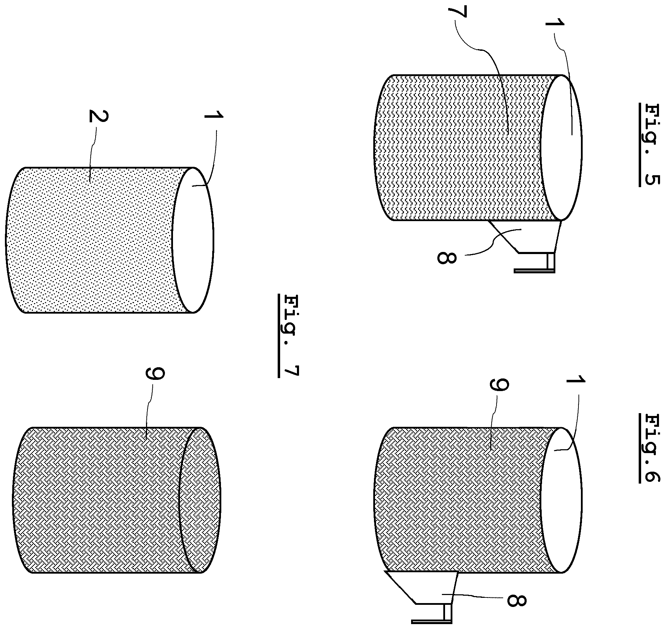

Dazu wird das Objekt 1 bevorzugt aus der geschlossenen Umgebung 3 entnommen. In Fig. 5 ist eine thermische Behandlung durch Bügeln dargestellt, bei welcher eine Hitzequelle 8 unter Ausübung von Druck über das Objekt 1 bewegt wird, um die Celluloseschicht 7 zu erhitzen und zu verdichten. Andere Optionen zur thermischen Behandlung wurden weiter oben bereits erwähnt.

Wie in Fig. 6 veranschaulicht ist, liegt am Ende der thermischen Behandlung ein stabilisiertes Cellulosegewebe 9 vor.

Wie in Fig. 7 veranschaulicht ist, wird das stabilisierte Cellulosegewebe 9 bevorzugt vom Objekt 1 abgenommen. Das Objekt 1 kann danach zur Bildung eines weiteren Cellulosegewebes 9 verwendet werden. Das Cellulosegewebe 9 liegt entsprechend der Form des Objekts 1 vor, auf dem es gewachsen ist. Das Cellulosegewebe 9 liegt insbesondere als ein in Umfangsrichtung geschlossener nahtloser Körper vor.

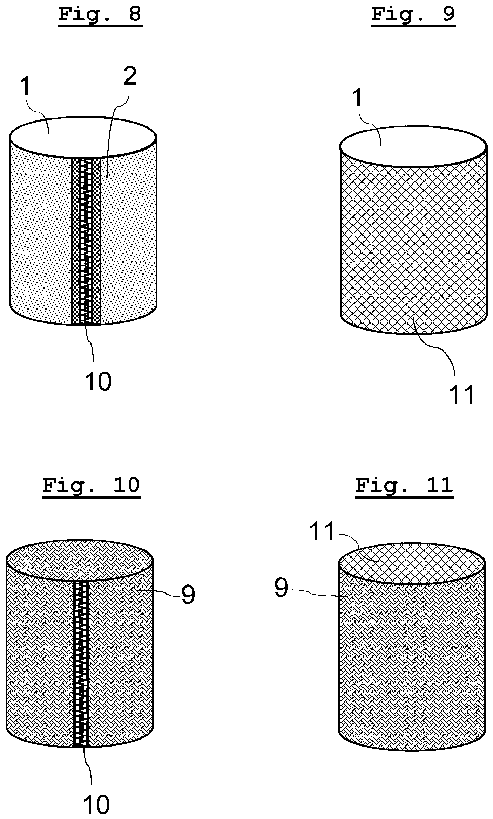

Fig. 8 veranschaulicht schematisch das Anbringen eines Gegenstandes 10 am Objekt 1, beispielsweise eines Reißverschlusses, bevor das Objekt 1 mit der Celluloseschicht 7 überwachsen wird. Wenn der Gegenstand 10 bzw. der Reißverschluss beidseits ein Stoffband aufweist, kann dieser in die Celluloseschicht integriert werden. Bereiche des Gegenstandes 10, welche nicht mit einer Celluloseschicht versehen werden sollen, können für die Dauer des Wachstumsprozesses abgedeckt werden, beispielsweise lackiert oder in Kunststoff eingeschweißt werden, und nach dem Wachstumsprozess wieder freigelegt werden.

Das resultierende Cellulosegewebe 9 mit einem eingewachsenen Gegenstand 10 ist in Fig. 10 veranschaulicht. Ein Gegenstand 10 kann in anderen Ausführungsvarianten auch gänzlich im Cellulosegewebe 9 integriert sein, beispielsweise in Form eines RFID-Tags.

In Fig. 9 ist veranschaulicht, dass auf dem Objekt 1 ein Gewebe 11 oder ein anderes Material aufgebracht werden kann, bevor das Objekt 1 mit der Celluloseschicht 7 überwachsen wird. Das Gewebe 11 oder ein anderes Material, wird dann von der Celluloseschicht umwachsen, zur dauerhaften Verbindung. Das Gewebe 11 oder ein anderes Material kann dabei die gesamte zu bewachsende Oberfläche des Objekts 1 bedecken oder nur bereichsweise aufgebracht werden.

Das resultierende Cellulosegewebe 9 mit einem umwachsenen Material, beispielsweise einem Gewebe 11, ist in Fig. 11 veranschaulicht. Das Material oder Gewebe 11 kann als Innenseite des Verbundmaterials vorliegen. Das Material oder Gewebe 11 kann auch allseits von Cellulosematerial umschlossen sein.

## Patentansprüche

1. Verfahren zur Bildung eines nahtlosen Cellulosegewebes (9), **gekennzeichnet durch** die Schritte,
dass in einem ersten Schritt zumindest eine Cellulose erzeugende Bakterien- und/oder Pilzkultur (4) auf die Oberfläche eines dreidimensionalen Objekts (1) aufgebracht wird,
dass in einem zweiten Schritt das dreidimensionale Objekt (1) in einer geschlossenen Umgebung (3) wiederholt mit einer Nährstofflösung (5) besprüht wird, solange bis die Bakterien- und/oder Pilzkultur (4) eine Celluloseschicht (7) in der gewünschten Dicke abgeschieden hat,
dass in einem dritten Schritt die Celluloseschicht (7) am dreidimensionalen Objekt thermisch bearbeitet wird, um das Cellulosegewebe (9) in stabilisierter Form zu erhalten.

2. Verfahren nach Anspruch 1, **dadurch gekennzeichnet, dass** zumindest ein Bereich der Oberfläche des dreidimensionalen Objekts (1) eine strukturierte Oberfläche (2) ist, insbesondere eine nanostrukturierte oder mikrostrukturierte Oberfläche ist.

3. Verfahren nach einem der Ansprüche 1 bis 2, **dadurch gekennzeichnet, dass** die geschlossene Umgebung (3) ein Reinraum oder ein Inkubator ist.

4. Verfahren nach einem der Ansprüche 1 bis 3, **dadurch gekennzeichnet, dass** in der geschlossenen Umgebung (3) zumindest ein Zuführmittel (6) zum automatisierten Besprühen des Objekts (1) vorliegt.

5. Verfahren nach einem der Ansprüche 1 bis 4, **dadurch gekennzeichnet, dass** die thermische Bearbeitung durch Einwirkung von Druck und Hitze erfolgt.

6. Verfahren nach einem der Ansprüche 1 bis 5, **dadurch gekennzeichnet, dass** nach dem dritten Schritt das Cellulosegewebe (9) vom Objekt (1) abgelöst wird.

7. Verfahren nach einem der Ansprüche 1 bis 6, **dadurch gekennzeichnet, dass** am Objekt (1) ein Gegenstand (10) oder ein Material platziert wird, welches im zweiten Schritt des Verfahrens mit Cellulosefasern überwachsen wird und dadurch in die Celluloseschicht (7) und somit in das Cellulosegewebe (9) integriert wird.

8. Verfahren nach einem der Ansprüche 1 bis 7, **dadurch gekennzeichnet, dass** die Dicke der Celluloseschicht (7) unterschiedlich stark ausgebildet wird, indem Bereiche des Objekts (1) unterschiedlich stark mit Nährstofflösung (5) versorgt werden.

9. Verfahren nach einem der Ansprüche 1 bis 8, **dadurch gekennzeichnet, dass** das Cellulosegewebe (9) als ein in Umfangsrichtung geschlossener nahtloser Körper vorliegt.
